(19) Europäisches Patentamt
European Patent Office
Office européen des brevets

(11) Publication number: 0 605 059 A1

# (12) EUROPEAN PATENT APPLICATION

(21) Application number: 93203708.8

(22) Date of filing: 27.12.93

(51) Int. Cl.[5]: C07D 401/04, A01N 43/50, A01N 43/54

(30) Priority: 31.12.92 EP 92311904

(43) Date of publication of application: 06.07.94 Bulletin 94/27

(84) Designated Contracting States: AT BE CH DE DK ES FR GB GR IE IT LI LU NL PT SE

(71) Applicant: SHELL INTERNATIONALE RESEARCH MAATSCHAPPIJ B.V.
Carel van Bylandtlaan 30
NL-2596 HR Den Haag(NL)

(72) Inventor: Munro, David
139 London Road
Maidstone, Kent ME16 0HF(GB)
Inventor: Patel, Bipin
8 Lyndhurst Grove
Sittingbourne, Kent ME10 4JA(GB)

(74) Representative: Allam, Peter Clerk et al
LLOYD WISE, TREGEAR & CO.
Norman House
105-109 Strand
London WC2R 0AE (GB)

## (54) Nitromethylene compounds and their use as pesticides.

(57) Nitromethylene compounds of general formula

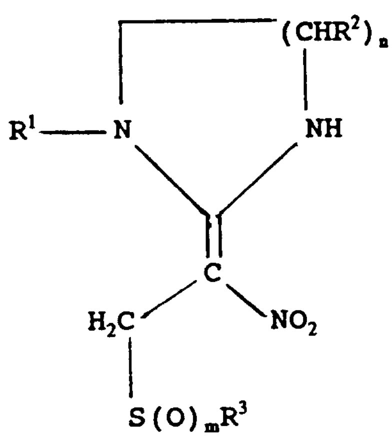

I

wherein n is 1 or 2; $R^1$ is an optionally substituted 3-pyridyl group; each $R^2$ is independently selected from an alkyl group, a haloalkyl group or a hydrogen atom; m is 0, 1 or 2; and $R^3$ is an optionally substituted aryl, alkyl, alkenyl or alkylcarbonyl group, processes for their preparation and their uses as pesticides are described.

EP 0 605 059 A1

This invention relates to nitromethylene compounds, particularly nitromethylene heterocyclic compounds, to processes for their preparation and to the use of such compounds as pesticides.

European Patent Application No. 0 369 526 (Shell) discloses nitromethylene compounds of general formula

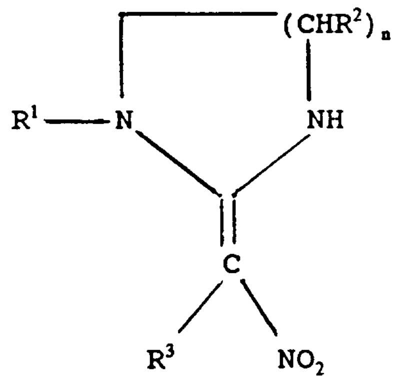

wherein n is 1, 2 or 3; $R^1$ is an optionally substituted 3-pyridyl group; each $R^2$ is independently selected from an alkyl group, a haloalkyl group or a hydrogen atom; and $R^3$ is a hydrogen atom or an alkyl carbonyl group.

This invention is based on the discovery of a novel class of nitromethylene compounds which exhibit pesticidal activity.

According to the present invention, there is provided nitromethylene compounds of general formula

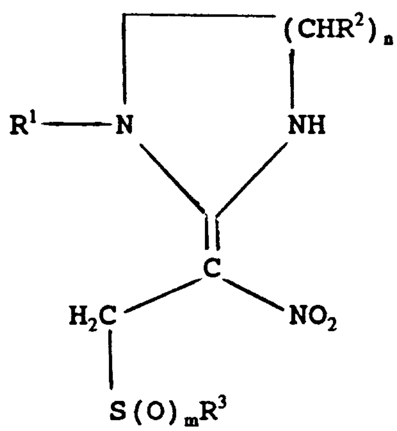

I

wherein n is 1 or 2; $R^1$ is an optionally substituted 3-pyridyl group; each $R^2$ is independently selected from an alkyl group, a haloalkyl group or a hydrogen atom; m is 0, 1 or 2; and $R^3$ is an optionally substituted aryl, alkyl, alkenyl or alkylcarbonyl group.

Unless otherwise stated in this specification, an alkyl or alkenyl group may be linear or branched and suitably contains up to 10, preferably up to 6, and most preferably up to 4, carbon atoms, preferred examples being methyl and ethyl.

Unless otherwise stated in this specification, when any groups are designated as being optionally substituted, the substituent groups which are optionally present may be any of those customarily employed in the development of pesticidal compounds, and/or the modification of such compounds to influence their structure/activity, persistence, penetration or other property. In relation to an alkyl or alkenyl group, specific examples of such substituents may include halogen, especially fluorine, chlorine or bromine atoms, and phenyl, alkoxy, hydroxy, cyano and (alkyl)amino groups. In relation to an aryl moiety, optional substituents may include halogen atoms, for example fluorine, chlorine, bromine and iodine atoms, and nitro, cyano, alkoxy, hydroxy, (alkyl)amino, alkyl and haloalkyl (especially $CF_3$) groups.

Examples of optional substituents on a 3-pyridyl group include halogen atoms and alkyl, alkoxy, alkylthio, haloalkyl, cyano, alkoxycarbonyl, alkylamino, dialkylamino, (alkylcarbonyl)alkylamino, (alkoxycarbonyl) alkylamino, alkylcarbonylamino and alkoxycarbonylamino groups. Any alkyl moiety in such substituents is preferably $C_{1-6}$ alkyl, more preferably $C_{1-4}$ alkyl.

$R^1$ is preferably a 3-pyridyl group substituted in the 6-position by a halogen atom, a $C_{1-4}$ alkoxy group, a $C_{1-4}$ alkylthio group, a $C_{1-4}$ haloalkyl group, a cyano group or a ($C_{1-4}$ alkoxy)carbonyl group, more preferably a 3-pyridyl group substituted in the 6-position by a chlorine or bromine atom, a methoxy group, a di- or trifluoromethyl group, or a cyano group. Advantageously, $R^1$ is a 6-chloro-3-pyridyl group.

Alkyl moieties present in groups represented by $R^2$ are preferably $C_{1-6}$ alkyl, more preferably $C_{1-4}$ alkyl, with methyl and ethyl being especially preferred. Whilst any of the moieties represented by $R^2$ in the $-(CHR^2)_n-$ group in compounds of formula I in which n is 2 may be alkyl or haloalkyl, it is preferred that alkyl or haloalkyl moieties represented by $R^2$ in such compounds are attached to carbon atoms having an adjacent nitrogen atom. Each $R^2$ is preferably a hydrogen atom.

To date the most promising activity has been found in compounds of formula I in which n is 2.

m is preferably 0.

Preferred optional substituents of an optionally substituted aryl group are halogen atoms. The most preferred substituent of said aryl group is a chlorine atom. A preferred optionally substituted aryl group is optionally substituted phenyl.

Where $R^3$ represents an optionally substituted alkyl, alkenyl or alkylcarbonyl group, the alkyl group is preferably a $C_{1-4}$ alkyl, more preferably a $C_{1-2}$ alkyl. A preferred optionally substituted alkyl group is an optionally substituted ethyl group. The alkenyl group is preferably a $C_{2-6}$ alkenyl. The alkylcarbonyl group preferably contains up to 8, more preferably up to 6 carbon atoms. A preferred optionally substituted alkylcarbonyl group is an optionally substituted alkylalkanoate group.

Where $R^3$ represents an alkyl, alkenyl or alkylcarbonyl group, preferably said group is unsubstituted.

Preferably $R^3$ represents an optionally substituted aryl, preferably phenyl, group.

Those skilled in the art will appreciate the possibility of the compounds of formula I existing as isomers (cis- and trans-isomers) and as tautomers. All such isomers and tautomers and their mixtures are embraced by the present invention.

The invention further provides a process for the preparation of a compound of general formula I as defined above which comprises treating a compound of general formula

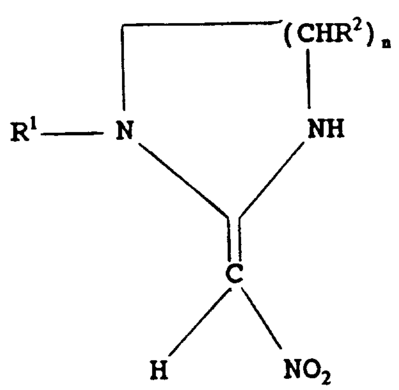

II

with formaldehyde and with a compound of general formula

$R^3S(O)_mH$ III; or

treating a compound of general formula

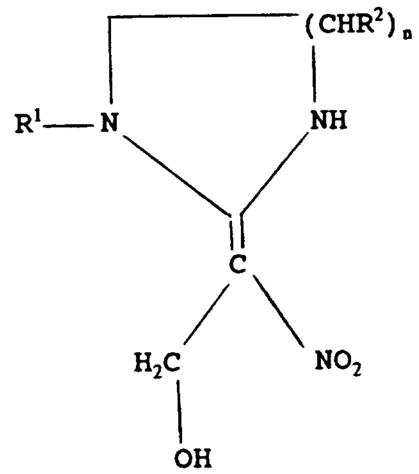

IV

with a compound of general formula

$R^3S(O)_mH$ III

wherein in each of compounds of general formula II, III and IV, n, $R^1$, $R^2$, $R^3$ and m are as defined above for compounds of the present invention.

Preferably, the preparation of the compound of general formula I starting from the compound of general formaul II involves mixing the compound of general formula II and formaldehyde together in the presence of an inert solvent. Suitable solvents include alcohols, for example, methanol or ethanol. Said compound of general formula III may then be added, suitably in the presence of a catalytic amount of concentrated acid, for example, in the presence of a small quantity of concentrated hydrochloric acid. The reaction is preferably carried out at a temperature in the range 10° to 60°C, more preferably, at ambient temperature, suitably with stirring. After subsequent removal of the solvent, the desired product may be isolated by standard procedures.

Preferably, the preparation of the compound of general formula I starting from the compound of general formula IV involves reacting compounds of general formula IV and III together in an inert solvent, for example, an alcohol, for example methanol or ethanol and in the presence of a catalytic amount of concentrated acid, for example, hydrochloric acid. The reaction is preferably carried out at a temperature within the range 10° to 60°C, more preferably, at ambient temperature, suitably with stirring. After removal of the solvent, the product may be isolated by standard procedures.

Compounds of general formula II may be prepared as described in European Patent Application No 0 369 526 (Shell). Compounds of general formula IV may be prepared by reacting a compound of general formula II with formaldehyde as described above. Compounds of general formula III are either known materials or may be prepared by standard procedures.

The compounds of general formula I exhibit pesticidal, particularly insecticidal, activity. Accordingly, the invention also provides a pesticidal composition comprising a carrier and, as active ingredient, a compound of general formula I. The invention further provides a method of combating pests at a locus, which comprises treating the locus with a pesticidal compound or composition according to the invention, and specifically provides the use as an insecticide of a compound of general formula I.

Particularly interesting activity has been observed against aphid pests. Further particularly interesting activity has been observed against mosquito pests. Preferred aspects of the present invention therefore relate to the pesticidal treatment of such pests.

A carrier in a composition according to the invention is any material with which the active ingredient is formulated to facilitate application to the locus to be treated, which may for example be a plant, seed or soil, or to facilitate storage, transport or handling. A carrier may be a solid or a liquid, including a material which is normally gaseous but which has been compressed to form a liquid, and any of the carriers normally used in formulating pesticidal compositions may be used. Preferably compositions according to the invention contain 0.5 to 95% by weight of active ingredient.

Suitable solid carriers include natural and synthetic clays and silicates, for example natural silicas such as diatomaceous earths; magnesium silicates, for example talcs; magnesium aluminium silicates, for example attapulgites and vermiculites; aluminium silicates, for example kaolinites, montomorillonites and

micas; calcium carbonate; calcium sulphate; ammonium sulphate; synthetic hydrated silicon oxides and synthetic calcium or aluminium silicates; elements, for example carbon and sulphur; natural and synthetic resins, for example coumarone resins, polyvinyl chloride, and styrene polymers and copolymers; solid polychlorophenols; bitumen; waxes; and solid fertilisers, for example superphosphates.

Suitable liquid carriers include water; alcohols, for example isopropanol and glycols; ketones, for example acetone, methyl ethyl ketone, methyl isobutyl ketone and cyclohexanone; ethers; aromatic or araliphatic hydrocarbons, for example benzene, toluene and xylene; petroleum fractions, for example kerosine and light mineral oils; chlorinated hydrocarbons, for example carbon tetrachloride, perchloroethylene and trichloroethane. Mixtures of different liquids are often suitable.

Agricultural compositions are often formulated and transported in a concentrated form which is subsequently diluted by the user before application. The presence of small amounts of a carrier which is a surface-active agent facilitates this process of dilution. Thus, preferably at least one carrier in a composition according to the invention is a surface-active agent. For example the composition may contain at least two carriers, at least one of which is a surface-active agent.

A surface-active agent may be an emulsifying agent, a dispersing agent or a wetting agent; it may be nonionic or ionic. Examples of suitable surface-active agents include the sodium or calcium salts of polyacrylic acids and lignin sulphonic acids; the condensation products of fatty acids or aliphatic amines or amides containing at least 12 carbon atoms in the molecule with ethylene oxide and/or propylene oxide; fatty acid esters of glycerol, sorbitan, sucrose or pentaerythritol; condensates of these with ethylene oxide and/or propylene oxide; condensation products of fatty alcohol or alkyl phenols, for example p-octylphenol or p-octylcresol, with ethylene oxide and/or propylene oxide; sulphates or sulphonates of these condensation products; alkali or alkaline earth metal salts, preferably sodium salts, of sulphuric or sulphonic acid esters containing at least 10 carbon atoms in the molecule, for example sodium lauryl sulphate, sodium secondary alkyl sulphates, sodium salts of sulphonated castor oil, and sodium alkylaryl sulphonates such as dodecylbenzene sulphonate; and polymers of ethylene oxide and copolymers of ethylene oxide and propylene oxide.

The compositions of the invention may for example be formulated as wettable powders, dusts, granules, solutions, emulsifiable concentrates, emulsions, suspension concentrates and aerosols. Wettable powders ususally contain 25, 50 or 75% w of active ingredient and usually contain in addition to solid inert carrier, 3-10% w of a dispersing agent and, where necessary, 0-10% w of stabiliser(s) and/or other additives such as penetrants or stickers. Dusts are ususally formulated as a dust concentrate having a similar composition to that of a wettable powder but without a dispersant, and are diluted in the field with further solid carrier to give a composition usually containing $^1/_2$-10% w of active ingredient. Granules are usually prepared to have a size between 10 and 100 BS mesh (1.676 - 0.152 mm), and may be manufactured by agglomeration or impregnation techniques. Generally, granules will contain $^1/_2$-75% w active ingredient and 0-10% w of additives such as stabilisers, surfactants, slow release modifiers and binding agents. The so-called "dry flowable powders" consist of relatively small granules having a relatively higher concentration of active ingredient. Emulsifiable concentrates usually contain, in addition to a solvent and, when necessary, co-solvent, 10-50% w/v active ingredient, 2-20% w/v emulsifiers and 0-20% w/v of other additives such as stabilisers, penetrants and corrosion inhibitors. Suspension concentrates are ususally compounded so as to obtain a stable, non-sedimenting flowable product and usually contain 10-75% w active ingredient, 0.5-15% w of dispersing agents, 0.1-10% w of suspending agents such as protective colloids and thixotropic agents, 0-10% w of other additives such as defoamers, corrosion inhibitors, stabilisers, penetrants and stickers, and water or an organic liquid in which the active ingredient is substantially insoluble; certain organic solids or inorganic salts may be present dissolved in the formulation to assist in preventing sedimentation or as antifreeze agents for water.

Aqueous dispersions and emulsions, for example compositions obtained by diluting a wettable powder or a concentrate according to the invention with water, also lie within the scope of the invention. The said emulsions may be of the water-in-oil or of the oil-in-water type, and may have a thick "mayonnaise"-like consistency.

Compositions in accordance with the invention may also contain other ingredients, for example other compounds possessing pesticidal, herbicidal, or fungicidal properties. The compounds of the invention may be found to be especially useful when applied in admixture with other insecticides and/or acaricides, e.g. organophosphates, pyrethroids, ureas and organotin compounds, for example the commercial products fenvalerate, permethrin, cypermethrin, deltamethrin, alphacypermethrin, fenbutatin oxide, flufenoxuron, diflubenzuron and trefluron.

The invention will be further understood from the following illustrative examples.

Example 1

Preparation of 1-(6-chloro-3-pyridyl)-2-(1-nitro-2-(3-chloro-phenylthio)ethylidene)imidazolidine.

[n = 1, $R^1$ = 6-chloro-3-pyridyl, $R^2$ = H, $R^3$ = 3-chlorophenyl, m = 0.]

Step 1

Preparation of N-(6-chloro-3-pyridyl)-1,2-diaminoethane.

This compound was prepared as described in Example 4 of European Patent Application Number 0 360 526.

Step 2

Preparation of 2-nitromethylene-1-(6-chloro-3-pyridyl) imidazolidine

N-(6-chloro-3-pyridyl)-1,2-diaminoethane (15g, 50%) prepared in Step 1 was dissolved in ethanol (30 ml) and added dropwise to a warm suspension of 1, 1-bis-(methylthio)-2-nitroethene (BSM) (12 g) in ethanol (100 ml). On addition, the slight deficit of BSM went into solution and methyl mercaptan was evolved. The reaction mixture was refluxed with stirring for about one hour until the evolution of gas ceased. A precipitate was deposited as the reaction mixture was allowed to cool overnight.

The product was filtered off and recrystallised to give 2-nitromethylene-1-(6-chloro-3-pyridyl) imidazolidine (10.6 g, 61%), m.p. 141 °C (MeOH).

Step 3

Preparation of 1-(6-chloro-3-pyridyl)-2-(1-nitro-2-(3-chloro-phenylthio)ethylidene)imidazolidine.

2-nitromethylene-1-(6-chloro-3-pyridyl)imidazolidine (2.5 g) prepared in step 2 was added to methanol (50 ml) and aqueous 40% formaldehyde (2.5 ml) was added. No clear solution was obtained even after 1 hour. 3-chlorothiophenol (1.5 ml) was then added with a drop of concentrated hydrochloric acid and the reaction mixture was stirred at ambient temperature overnight. All of the solvent was then removed and the residue dissolved in chloroform. TLC (silica; $CHC1_3$ 90%, MeOH 10%) indicated a complex mixture, the main spot having the same Rf as the starting mixture. Chromatography (as TLC) gave the title compound as a yellow solid (2.8 g, 68%). The product was recrystallised. m.p. 163 °C(EtOH).

| Analysis | | | |
|---|---|---|---|
| | %C | %H | %N |
| Calc. | 48.4 | 3.5 | 14.1 |
| Found | 48.4 | 3.7 | 14.0 |

Examples 2 to 15

Further compounds as noted in Table 1 were prepared in processes analogous to the process of Example 1. Characterising data for the compounds of Examples 2 to 15 is noted in Table 2.

TABLE 1

| EXAMPLE NO. | n | $R^1$ | $R^2$ | $R^3$ | m |
|---|---|---|---|---|---|
| 2 | 1 | 6-chloro-3-pyridyl | H | phenyl | 0 |
| 3 | 1 | 6-chloro-3-pyridyl | H | 2-chlorophenyl | 0 |
| 4 | 1 | 6-chloro-3-pyridyl | H | 4-chlorophenyl | 0 |
| 5 | 1 | 6-chloro-3-pyridyl | H | ethyl | 0 |
| 6 | 2 | 6-chloro-3-pyridyl | H | phenyl | 0 |
| 7 | 2 | 6-chloro-3-pyridyl | H | 3-chlorophenyl | 0 |
| 8 | 2 | 6-chloro-3-pyridyl | H | ethyl | 0 |
| 9 | 2 | 6-chloro-3-pyridyl | H | phenylmethyl | 0 |
| 10 | 2 | 6-chloro-3-pyridyl | H | 3-butenyl | 0 |
| 11 | 2 | 6-chloro-3-pyridyl | H | $-CH_2CO.OCH_2CH_3$ | 0 |
| 12 | 2 | 6-bromo-3-pyridyl | H | ethyl | 0 |
| 13 | 1 | 6-methoxy-3-pyridyl | H | phenyl | 0 |
| 14 | 2 | 6-methoxy-3-pyridyl | H | phenyl | 0 |
| 15 | 2 | 6-bromo-3-pyridyl | H | phenyl | 0 |

TABLE 2

| EXAMPLE NO. | ANALYSIS | | | | MP/°C |
|---|---|---|---|---|---|
| | %C | | %H | %N | |
| 2 | calc | 53.0 | 4.1 | 15.4 | 151 |
| | found | 52.6 | 4.3 | 15.5 | |
| 3 | calc | 48.4 | 3.5 | 14.1 | 181 |
| | found | 48.5 | 3.6 | 14.2 | |
| 4 | calc | 48.4 | 3.5 | 14.1 | 194 |
| | found | 48.2 | 3.7 | 14.0 | |
| 5 | calc | 45.8 | 4.8 | 17.8 | 148 |
| | found | 46.6 | 5.0 | 18.0 | |
| 6 | calc | 54.2 | 4.5 | 14.9 | 158 |
| | found | 53.9 | 4.5 | 14.7 | |
| 7 | calc | 49.6 | 3.9 | 13.6 | 148 |
| | found | 49.7 | 3.9 | 13.6 | |
| 8 | calc | 47.5 | 5.2 | 17.0 | 157 |
| | found | 47.4 | 5.2 | 16.8 | |
| 9 | calc | 55.3 | 4.9 | 14.3 | 146 |
| | found | 54.3 | 5.1 | 13.7 | |
| 10 | calc | 49.3 | 5.0 | 16.4 | 100 |
| | found | 48.5 | 5.3 | 16.3 | |
| 11 | calc | 46.6 | 4.9 | 14.5 | 87 |
| | found | 47.0 | 5.0 | 14.5 | |
| 12 | calc | 41.8 | 4.6 | 15.0 | 158 |
| | found | 42.0 | 4.6 | 15.0 | |
| 13 | calc | 57.0 | 5.0 | 15.7 | 182 |
| | found | 56.3 | 5.1 | 15.3 | |
| 14 | calc | 58.1 | 5.4 | 15.0 | 127 |
| | found | 57.9 | 5.4 | 15.1 | |
| 15 | | - | - | - | oil* |
| | | - | - | - | |

* identified by $^{1}H$ NMR

EXAMPLE 16

Pesticidal Activity

Pesticidal activity of compounds of the invention was assessed against various of the following pests:-
Spodoptera littoralis (Egyptian cotton leafworm)
Aedes aegypti (yellow fever mosquito)

Musca domestica (housefly)
Acyrthosiphon pisum (pea aphid)
Megoura viciae (vetch aphid)
Phaedon cochleariae (mustard beetle)
Trialeurodes vaporariorum (greenhouse whitefly)
Nephotettix cincticeps (green leaf hopper)
Nilaparvata lugens (brown rice plant hopper)

The test methods employed for each species appear below. In each test, unless otherwise stated, solutions or suspensions of test compound were made up over a range of concentrations in water (initially O.1%w) containing 10%w acetone and 0.025%w "TRITON X-100" (trade mark) surface active agent (the condensation product of ethylene oxide with an alkyl phenol). These solutions were sprayed at a rate equivalent to 340 litres per hectare ($3.4 \times 10^{-5} m^3/m^2$) onto Petri dishes containing either test species per se or diet onto which test species were subsequently introduced, as indicated. In some assays leaf discs infested with test species were sprayed whilst other assays involved the spraying of plants which were infested subsequently with test species after the spray solution had dried. The tests were all conducted under normal insectary conditions (23°C ± 2°C, fluctuating humidity and light). Mortality assessments were made as indicated below, in terms of percentage mortality figures. In each test a $LC_{50}$ (the dosage of active material required to kill half of the test species) for the compound was calculated from the mortality figures and compared with the corresponding $LC_{50}$ for a standard insecticide, ethyl parathion, in the same test. The results are expressed as toxicity indices thus:

$$\text{toxicity index} \quad \frac{LC_{50}\ (\text{parathion})}{LC_{50}\ (\text{test compound})} \times 100$$

(i) Spodoptera littoralis (7 day)(S1 7D)

Test solutions were sprayed as indicated above onto Petri dishes containing a nutritious diet for Egyptian cotton leafworm larvae. When the spray deposit had dried, each dish was infested with ten 2nd instar larvae. Mortality assessments were made 7 days after spraying.

(ii) Spodoptera littoralis (foliar)(S1 Fol)

Test solutions were sprayed as described above onto Petri dishes containing 9cm discs of Chinese cabbage leaves on filter papers. After drying, each dish was infested with ten 2nd instar larvae. Mortality assessments were made 24 hours after infestation.

(iii) Spodontera littoralis (ovicidal)(S1 OA)

Test solutions were sprayed as described above onto Petri dishes containing filter papers on which were approximately 50 24 hour old eggs. After 6 days the numbers of hatched and unhatched eggs were counted and percentage mortality calculated.

(iv) Aedes aegypti (Aa)

Early 4th instar larvae were used. Test solutions were made up to 0.5 ppm of test compound (and progressive half-dilutions) in water containing 0.04%w "TRITON X-100" (trade mark); acetone was initially present to aid solution, but was allowed to evaporate off before introduction of larvae.

Ten early 4th instar larvae were placed in 100 ml of test solution held at 28°C, and after 48 hours, larval mortality was recorded. The final mortality was assessed by counting the number of emerged adult mosquitoes after one week.

(v) Musca domestica (Md)

Batches of ten 2 to 3 day old milk-fed adult female houseflies, anaesthetised using carbon dioxide, were placed on filter papers inside Petri dishes. The dishes were sprayed with the test solutions as described above. The flies were retained in the Petri dishes and were fed with a dilute milk solution which was dripped down the side of the Petri dish and absorbed by the filter paper. Mortality was assessed after 24 hours.

(vi) Acyrthosiphon pisum (Ap)

Tests were carried out on young adult pea aphids. Whole pea plants 6 days after germination were placed on filter papers in Petri dishes. Ten aphids were transferred to each pea plant and left for 30 minutes to allow the aphids to settle and start to feed. The dishes were then sprayed with the test solutions as described above and lids were placed on the Petri dishes. Mortality was assessed after 24 hours.

(vii) Megoura viciae (My)

Tests were carried out on adult-Vetch aphids. Pairs of broad bean leaves on filter paper in Petri dishes were sprayed side by side with uncounted quantities of aphids in small gauze-covered containers. After passing through the spray ten aphids were tipped onto the leaves and lids were placed on the Petri dishes. Mortality was assessed after 24 hours.

(viii) Phaedon cochleariae (Pc)

Test solutions were sprayed as described above onto Petri dishes containing 9cm discs of Chinese cabbage leaves on filter papers. After drying, each dish was infested with ten adult mustard beetles (up to 1 week old). Mortality assessments were made 24 hours after infestation.

(ix) Trialeurodes vaporariorum (Tv)

French bean plants (Phaseolus vulgaris) with two fully expanded leaves were placed in a breeding culture of T. vaporariorum, also on French bean plants, which were then disturbed to ensure resettlement on the introduced plants. During the subsequent 24 hour period, eggs were deposited and kept at 27°C, with 14 hours photoperiod. All adult whiteflies were then carefully removed, leaving egg samples of a known age. After eight days the majority of eggs had hatched. Leaf discs containing the newly hatched nymphs were then cut from the leaves and transferred to moist filter paper. The discs were examined under a low-powered microscope to determine the exact number of 1st instar nymphs per disc and to remove any unhatched eggs. On average, 70-100 nymphs were found per disc.

The discs were transferred into Petri dishes and sprayed with test solutions as described above. After 6 days percentage mortalities were assessed.

(x) Nephotettix cincticeps (Nc)

Tests were carried out on young adult female green leaf hoppers. Plant pots, each containing five rice seedlings 10 to 15 cm tall arranged across the centre of the pot, were sprayed with test solutions as described above (but initial test concentration 0.05% of test compound). Spraying was on both sides of the plants with the pots horizontal. One hour after spraying, each pot was filled to the brim with fine silver sand, an open-ended glass jar was placed over each pot and each pot was infested with ten hoppers. A paper tissue was placed over the open end of each glass jar to retain the hoppers. The pots were irrigated from underneath, maintained at a temperature of 27°C ± 2°C and subjected to white fluorescent light under a regime of 18 hours light followed by 6 hours darkness. Mortality assessments were made 48 hours after infestation.

(xi) Nilaparvata lugens (Nl)

Tests were carried out on young adult female brown rice plant hoppers in the same way as for green leaf hoppers in (x) above.

## EXAMPLE 17

### Acaricidal Activity (ovicide)(Tu OA)

Acaricidal activity of the compounds of Examples 1 to 15 was assessed, employing eggs of the glasshouse red spider mite, Tetranychus urticae (T.u.), less than 24 hours old, by the following procedure.

2cm diameter leaf discs cut from the leaves of French bean plants were placed on filter paper, kept moist by a cotton wool wick dipped into water.

On the day before spraying, each leaf disc was infested with 10 female adult mites. On the day of the test, the adults were removed, leaving the eggs laid overnight on the discs. The leaf discs were then sprayed with solutions of test compound made up as in Example 16 above, at a rate equivalent to 340 litres per hectare ($3.4 \times 10^{-5}$ $m^3/m^2$).

Throughout the test, the eggs were held under normal insectary conditions ( 23°C ± 2°C, fluctuating humidity and 16 hours days length). After 7-10 days, the numbers of hatched nymphs and unhatched eggs were assessed and the percentage mortality calculated. The $LC_{50}$ (the dosage of active material required to kill half of the test species) for the compound was calculated from the mortality figure and compared with the corresponding $LC_{50}$ for a standard insecticide, chlorfenson, in the same test. The result is expressed as toxicity index thus:

$$\text{toxicity index} = \frac{LC_{50}\ (\text{chlorfenson})}{LC_{50}\ (\text{test compound})} \times 100$$

Results of the assessments described in Examples 16 and 17 for each of the compounds of Examples 1 to 15 are provided in Table 3. In the Table, grades B and C indicate mortalities of 40-69% and 0-39%, respectively, at the initial test concentration of O.1%w (1000 ppm). For compounds achieving a mortality of 70-100%, mortality assessments were made as indicated above, to yield the toxicity indices shown by the figures in the Table.

**TABLE 3**

| Compound of Example | Toxicity Indices | | | | | | | | | | | |
|---|---|---|---|---|---|---|---|---|---|---|---|---|
| | Sl7D | SlFol | SlOA | Aa | Md | Ap | Mv | Pc | Tv | Nc | Nl | TuOA |
| 1 | C | C | | 120 | C | 13 | 0 | B | B | 140 | - | C |
| 2 | C | C | C | 67 | <1 | 100 | 4.5 | 9 | B | 200 | - | C |
| 3 | C | C | C | 35 | C | 3 | 0 | B | B | 45 | - | C |
| 4 | C | C | C | 21 | C | C | 0 | 4 | C | 26 | - | C |
| 5 | C | C | C | 5 | C | C | 0 | 10 | B | 380 | - | C |
| 6 | C | C | C | 18 | C | 90 | 0 | 46 | B | 1300 | - | C |
| 7 | C | C | - | 13 | <2 | 160 | 4 | 160 | 610 | 500 | - | C |
| 8 | C | C | 17 | 6 | C | 25 | 2.5 | 91 | 300 | 1900 | 15000 | C |
| 9 | C | C | C | 10 | 2 | 29 | 3.5 | - | B | 1000 | 3500 | C |
| 10 | C | C | C | 9 | C | 26 | 0 | - | 15 | 2500 | - | C |
| 11 | C | C | C | 19 | C | 32 | 0 | - | B | 1300 | - | C |
| 12 | C | C | C | 3 | C | 24 | 2 | 100 | 270 | C | - | C |
| 13 | C | C | C | <4 | C | <2 | 0 | C | C | C | - | C |
| 14 | C | C | C | <4 | C | 3 | 0 | 10 | C | 58 | - | C |
| 15 | C | C | C | 12 | C | 72 | 1.5 | 4.7 | B | 670 | 4200 | C |

## Claims

**1.** Nitromethylene compounds of general formula

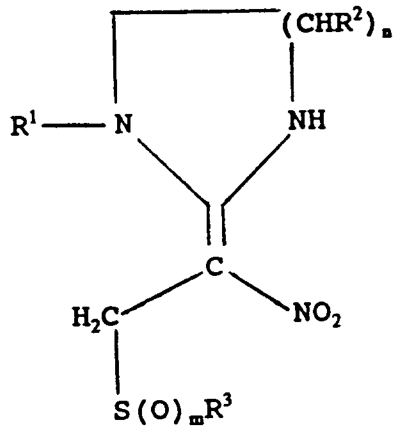

I

wherein n is 1 or 2; $R^1$ is an optionally substituted 3-pyridyl group; each $R^2$ is independently selected from an alkyl group, a haloalkyl group or a hydrogen atom; m is 0, 1 or 2; and $R^3$ is an optionally substituted aryl, alkyl, alkenyl or alkylcarbonyl group.

**2.** A compound as claimed in claim 1, wherein $R^1$ is a 3-pyridyl group substituted in the 6-position by a halogen atom, a $C_{1-4}$ alkoxy group, a $C_{1-4}$ alkylthio group, a $C_{1-4}$ haloalkyl group, a cyano group or a ($C_{1-4}$ alkoxy)carbonyl group.

**3.** A compound as claimed in claim 1 or claim 2, wherein $R^2$ represents a hydrogen atom.

**4.** A compound as claimed in any preceding claim, wherein m is 0.

**5.** A compound as claimed in any preceding claim, wherein $R^3$ represents an optionally substituted aryl group.

**6.** A process for the preparation of a compound of general formula I as claimed in any preceding claim which comprises treating a compound of general formula

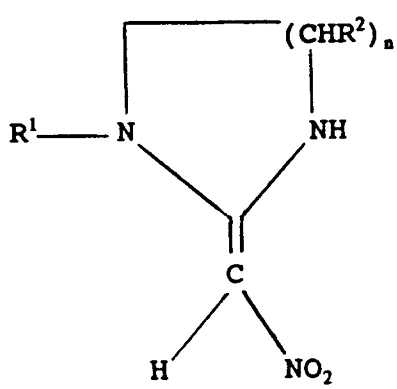

II

with formaldehyde and with a compound of general formula

$R^3S(O)_mH$ III; or

treating a compound of general formula

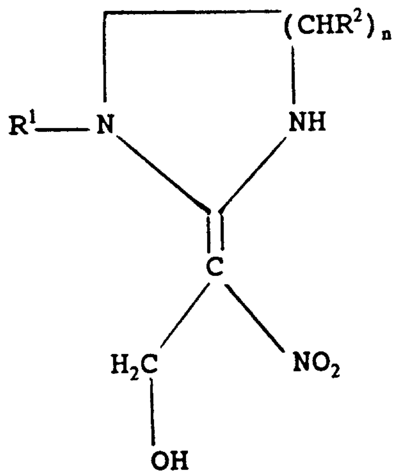

IV

with a compound of general formula

$R^3S(O)_mH$ III

wherein in each of the compounds of general formula II, III and IV, n, $R^1$, $R^2$, $R^3$ and m are as defined in any preceding claim.

**7.** A pesticidal composition comprising a carrier and, as active ingredient, a compound of general formula I as claimed in any of claims 1 to 5.

**8.** A method of combating pests at a locus, which comprises treating the locus with a pesticidal compound or composition as claimed in any of claims 1 to 5 or 7.

**9.** Use as an insecticide of a compound of general formula I as claimed in any of claims 1 to 5.

European Patent Office

# EUROPEAN SEARCH REPORT

Application Number
EP 93 20 3708

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| D,Y | EP-A-0 369 526 (SHELL)<br>* claims 1,8,10 * | 1,7,9 | C07D401/04<br>A01N43/50<br>A01N43/54 |
| Y | EP-A-0 392 560 (TAKEDA)<br>* claims 1,14; examples 8,13,14; table 3 * | 1,7,9 | |
| A | EP-A-0 292 822 (BAYER)<br>* claims 1,5,6 * | 1,7,9 | |
| A | EP-A-0 437 784 (ISHIHARA SANGYO KAISHA)<br>* claims 1,13 * | 1,7,9 | |

TECHNICAL FIELDS SEARCHED (Int.Cl.5)

C07D
A01N

The present search report has been drawn up for all claims

| Place of search | Date of completion of the search | Examiner |
|---|---|---|
| THE HAGUE | 18 April 1994 | Voyiazoglou, D |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document